# EUROPEAN PATENT SPECIFICATION

(11) **EP 0 651 653 B1**
(45) Date of publication and mention of the grant of the patent: **10.03.2004**
(21) Application number: 93918436.2
(22) Date of filing: 22.07.1993
(51) Int. Cl.: A61K 39/39, A61K 39/00, C12P 21/02

(54) **p53 VACCINE**
P53 IMPFSTOFF
VACCIN p53

(30) Priority: 22.07.1992 US 918292; 09.02.1993 US 15493
(43) Date of publication of application: 10.05.1995
(60) Divisional application: 03028489.7
(73) Proprietor: THE TRUSTEES OF PRINCETON UNIVERSITY, Princeton, NJ 08544-0036 (US)
(72) Inventor: LEVINE, Arnold, J., Princeton, NJ 08540 (US)
(74) Representative: Grünecker, Kinkeldey, Stockmair & Schwanhäusser Anwaltssozietät
(86) International application number: PCT/US1993/007069
(87) International publication number: WO 1994/002167

(56) References cited:
- WO-A-91/13632
- WO-A-93/10814
- WO-A-93/24525
- J. EXP. MED., vol. 172, August 1990, pages 631-640, XP000569394 INABA ET AL.: "Dendritic cells pulsed with protein antigens in-vitro can prime antigen-specific, MHC-restricted T cells in situ"
- CURR. OPIN. ONCOL., vol. 4, no. 3, June 1992, pages 435-441, XP000569586 OZEN H.: "Bladder cancer"
- Nature, Volume 351, issued 06 June 1991, A.J. LEVINE et al., "The p53 Tumour Suppressor Gene", pages 453-456, see entire document.
- Nature, Volume 351, issued 06 June 1991, C.K. STOVER et al., "New Use of BCG for Recombinant Vaccines", pages 456-460, see entire document.
- Vaccines 91, issued 1991, C.K. STOVER et al., "Development of BCG as a Live Recombinant Vaccine Vehicle", pages 393-398, see entire document.
- Proceedings of the National Academy of Sciences, Volume 87, issued January 1990, A. KASID et al., "Human Gene Transfer: Characterization of Human Tumor Infiltrating Lymphocytes as Vehicles for Retroviral-Mediated Gene Transfer in Man", pages 473-477, see entire document.
- Proceedings of the National Academy of Sciences, Volume 88, issued April 1991, K. CULVER et al., "Lymphocytes as Cellular Vehicles for Gene Therapy in Mouse and Man", pages 3155-3159, see entire document.
- Journal of Experimental Medicine, Volume 169, issued April 1989, S.E. MACATONIA et al., "Primary Stimulation by Dendtritic Cells Induces Antiviral Proliferative and Cytotoxic T Cell Responses in Vitro", pages 1255-1264, see entire document.
- D.P. STITES et al., "Basic and Clinical Immunology", published 1991, by APPLETON & LANGE (CONN.), see pages 580-587.
- Journal of Experimental Medicine, Volume 173, issued January 1991, S. JUNG et al., "Human T Lymphocyte Recognize a Peptide of Single Point-Mutated, Oncogenic Ras Proteins", pages 273-276, see entire document.
- Science, Volume 250, issued 05 October 1990, O. HALEVY et al., "Different Tumor-Derived p53 Mutants Exhibit Distinct Biological Activities", pages 113-116, see entire document.
- Science, Volume 249, issued 24 August 1990, S.J. BAKER et al., "Suppression of Human Colorectal Carcinoma Cell Growth by Wild-Type p53", pages 912-915, see entire document.

## Description

The present invention is directed to a vaccine for treating cancer. The vaccine comprises a p53 protein as the immunogen.

The p53 gene, which is found on chromosome 17p of the human genome, is a tumor suppressor gene in its wild-type state. A review article by Levine et al. entitled "The p53 Tumor Suppressor Gene" appears in Nature 351, 453-456 (1991).

More than 50% of human tumors contain cells expressing a mutant form of the p53 gene. In many tumors, one allele of the p53 gene contains a point mutation that encodes a mutant form of the protein while the other allele is partially or totally lost. This pattern is observed, for example, in approximately 70-80% of colon cancers, 50% of breast cancers, and 50% of lung cancers including 100% of small cell lung cancers. Suggestions have been made to diagnose cancers by detecting the loss of wild type p53 (see Vogelstein et al., European Patent Application 390,323 and Baker et al., Science 244, 217-221 (1989).

The position or location of the point mutation in the p53 gene differs in different types of tumors. For example, 50% of the hepatocellular carcinomas in humans exposed to hepatitis B and aflotoxin contain p53 mutations at codon 249; lung tumors appear to contain mutations preferentially at codons 154 and 273; colon tumors have multiple independent mutations at codons 175, 248, and 273. Evidence has been presented that various phenotypes, including the severity and nature of cancer and pre-cancer states, can be distinguished by determining the site of p53 mutations. See Levine et al., International Application No. PCT/US91/04608, filed June 27, 1991.

Approximately 10-20% of humans with cancers have tumors that produce antibodies directed against the p53 protein; de Fromentel et al., International Journal of Cancer 39, 185-189 (1987); Crawford et al., International Journal of Cancer 30, 403-408 (1982). The presence of these antibodies suggests that class II receptors of the human HLA or the murine H-2 locus can present peptide antigens of p53 to the CD-4 helper T-cell and B-cell system, resulting in an immune response. Antibodies are not, however, believed to be effective anti-tumor agents. Therefore, the presence of anti-p53 antibodies in humans with cancer does not suggest the possibility of cancer patients producing an effective anti-tumor immune response.

There are reports that animals immunized with a tumor antigen are protected against the same antigen. Thus, immunizing animals with simian virus 40 (SV40) large T antigen can protect against subsequent challenges with live tumorigenic SV40-transformed cells; see Tevethia et al., Cold Spring Harbor Symp. Quant. Biol. 44, 235-242 (1980).

Similarly, Frey and Levine have reported that rats immunized with an irradiated p53-plus-ras-transformed Fisher rat cell line, designated B3, were protected from subsequent tumor challenge with the same live cell. The p53-plus-ras-transformed rat cell lines were reported to express a tumor-specific transplantation rejection antigen that is common to 85% of independently derived p53-plus-ras-transformed cell lines. Frey and Levine presented evidence that the p53 protein is not the tumor-specific transplantation rejection antigen, and does not protect against challenge by B3 cells; see Journal of Virology 63, 5440-5444 (1989).

Current cancer treatments involve cytotoxic agents, such as chemical compounds and radiation, that are insufficiently specific to tumor cells. There is a need for more specific treatments that do not affect normal cells. There is a particular need for cancer treatments that result from stimulating a patient's own immune system.

### SUMMARY OF THE INVENTION

These and other objects as will be apparent to those having ordinary skill in the art have been met by providing a vaccine composition comprising a wild-type p53 protein in a form that, when presented to the immune system of a mammal, induces an effective immune response.

The invention further provides a method of inhibiting the growth of tumors in mammals comprising treating a mammal with an immunologically effective amount of a wild-type p53 protein.

### DETAILED DESCRIPTION OF THE INVENTION

The subject invention provides a vaccine composition comprising a wild-type p53 protein in a form that, when presented to the immune system of a mammal, induces an effective immune response. For example, the mutant or wild-type p53 protein may be present on the surface of an antigen presenting cell or liposome, or combined with a pharmaceutically acceptable adjuvant.

For the purposes of the present specification, the term "wild-type p53 protein" means the nucleotide or amino acid sequence reported by Matlashewski et al, EMBO J. 13, 3257-3262 (1984); Zakut-Houri et al, EMBO J. 4, 1251-1255 (1985); and Lamb and Crawford, Mol. Cell. Biol. 5, 1379-1385 (1986). The sequences are available from GenBank. Wild-type p53 includes a proline/arginine polymorphism at amino acid 72 and the corresponding nucleotide polymorphism.

The p53 protein may be mutated. The mutation is preferably at a position that is frequently found to be mutated in tumor cells, and that leads to inactivation of the wild-type p53 gene. The mutations may be either a single substitution or multiple (i.e. 2-20, preferably 2-10, more preferably 2-5) substitutions.

Suitable mutant human p53 genes are described in Levine, A.J. et al., The p53 Tumor Suppressor Gene, Nature 351:453-456 (1991). Most of the point mutations that occur in the p53 gene are missense mutations, giving rise to an altered p53 protein. The majority of mutations are clustered between amino-acid residues 130 and 290, and mostly localized in four "hot spot" regions of the protein, which coincide with the four most highly conserved regions of the p53 gene; see Nigro et al, Nature 342, 705-708 (1989). The four "hot spot" mutation regions are at codons 132-143; 174-179; 236-248; and 272-281. The frequency and distribution of these hot spots differ among cancers from different tissue types.

The wild-type p53 gene and protein are known, and may be obtained in natural or recombinant form by known methods. Such methods include isolating the protein directly from cells; isolating or synthesizing DNA encoding the protein and using the DNA to produce recombinant protein; and synthesizing the protein chemically from individual amino acids. Methods for obtaining the wild-type p53 gene and protein are described in Matlashewski et al, EMBO J. 13, 3257-3262 (1984); Zakut-Houri et al, EMBO J. 4, 1251-1255 (1985); and Lamb and Crawford, Mol. Cell. Biol. 5, 1379-1385 (1986). Mutants may be prepared from the wild-type p53 gene by site-directed mutagenesis; see, for example, Zoller and Smith, Nucl. Acids Res. 10, 6487-6500 (1982); Methods in Enzymology 100, 468-500 (1983); and DNA 3, 479-488 (1984).

The entire p53 gene or fragments of the p53 gene may, for example, be isolated by using the known DNA sequence to construct oligonucleotide probes. To do so, DNA restriction fragments are identified by Southern hybridization using labelled oligonucleotide probes derived from the known sequence.

Alternatively, p53-encoding DNA may be synthesized from individual nucleotides. Known methods for synthesizing DNA include preparing overlapping double-stranded oligonucleotides, filling in the gaps, and ligating the ends together.

The DNA prepared as described above may be amplified by polymerase chain reaction (PCR). Alternatively, the DNA may be amplified by insertion into a cloning vector, which is transfected into a suitable host cell, from which the p53 DNA may be recovered. See, generally, Sambrook et al, "Molecular Cloning," Second Edition, Cold Spring Harbor Laboratory Press (1987).

Recombinant methods well known in the art may be used for preparing the protein. Briefly, p53-encoding DNA is inserted into an expression vector, which is transfected into a suitable host. The DNA is expressed, and the protein is harvested. See Sambrook et al., Id.

Equivalents of the wild-type p53 protein may also be used in the vaccine of the invention. Such equivalents include analogs that induce an immune response comparable to that of the wild-type p53 protein. In addition, such equivalents are immunologically cross-reactive with their corresponding mutant or wild-type p53 protein. The equivalent may, for example, be a fragment of the protein, or a substitution, addition or deletion mutant of the mutant or wild-type p53 protein.

The wild-type p53 protein fragment preferably contains sufficient amino acid residues to define an epitope of the antigen. The fragment may, for example, be a minigene encoding only the epitope. Methods for isolating and identifying immunogenic fragments from known immunogenic proteins are described by Salfeld et al. in J. Virol. 63, 798-808 (1989) and by Isola et al. in J. Virol. 63, 2325-2334 (1989).

If the fragment defines the epitope, but is too short to be immunogenic, it may be conjugated to a carrier molecule. Some suitable carrier molecules include keyhole limpet hemocyanin, Ig sequences, TrpE, and human or bovine serum albumen. Conjugation may be carried out by methods known in the art. One such method is to combine a cysteine residue of the fragment with a cysteine residue on the carrier molecule.

Equivalent proteins have equivalent amino acid sequences. An amino acid sequence that is substantially the same as another sequence, but that differs from the other sequence by one or more substitutions, additions and/or deletions, is considered to be an equivalent sequence. Preferably, less than 25%, more preferably less than 10%, and most preferably less than 5% of the number of amino acid residues in a sequence are substituted for, added to, or deleted from the proteins of the invention.

For example, it is known to substitute amino acids in a sequence with equivalent amino acids. Groups of amino acids generally considered to be equivalent are:
(a) Ala(A) Ser(S) Thr(T) Pro(P) Gly(G);
(b) Asn(N) Asp(D) Glu(E) Gln(Q);
(c) His(H) Arg(R) Lys(K);
(d) Met(M) Leu(L) Ile(I) Val(V); and
(e) Phe(F) Tyr(Y) Trp(W).

The wild-type p53 protein of the invention unexpectedly induces an effective immune response when properly presented to the immune system. For the purposes of this specification, an effective immune response inhibits, i.e. prevents, slows or stops, the growth of cancer cells, or eliminates cancer cells. The effective immune response is preferably a killer T-cell response. The mammal may be a human or animal typically used for experimentation, such as mice, rats or rabbits.

The wild-type p53 is presented to the immune system as a vaccine by a vehicle. For example, the wild-type p53 may be present on the surface of an antigen presenting cell or combined with a pharmaceutically acceptable adjuvant.

Antigen presenting cells are generally eukaryotic cells with major histocompatibility complex (MHC), preferably Class II, gene products at their cell surface. For the purposes of this specification, antigen presenting cells also include recombinant eucaryotic cells such as peripheral blood cells and recombinant bacterial cells. Some examples of antigen presenting cells as defined by this specification include dendritic cells, macrophages that are preferably MHC Class II positive, monocytes that are preferably MHC Class II positive, and lymphocytes.

In one embodiment of the subject invention, the antigen presenting cell is a recombinant eucaryotic cell that expresses exogenous DNA encoding mutant or wild-type p53 protein. The recombinant eucaryotic cell may be prepared in vivo or in vitro.

In one embodiment, DNA encoding wild-type p53 is inserted into the eucaryotic cell in vivo using recombinant viral vectors. These vectors include an attenuated recombinant poxvirus, such as vaccinia virus, preferably parrot pox, that has its nonessential virus-encoded genetic functions inactivated, described in International Application WO 92/15672. Techniques for the insertion of foreign DNA into the vaccinia genome are known in the art. Plasmid vectors for the construction of recombinant viruses are described in, for example, Chakrabarti et al. (1985) Mol. Cell Biol. **5**:3403; Mackett et al., (1984), J. Virol. **49**:857; and Moss (1987), page 10 of Gene Transfer Vectors for Mammalian Cells, Miller and Calos, eds., Cold Spring Harbor Laboratory, Cold Spring Harbor, N.Y. Expression of the wild-type p53 protein then occurs in vivo in an antigen presenting cell in subjects immunized with the recombinant poxvirus.

In another embodiment, DNA encoding wild-type p53 is inserted into the eucaryotic cell in vitro using known techniques, such as the retroviral transduction techniques described for tumor infiltrating lymphocytes (TILs) (S.A. Rosenberg et al., NEJM, **323**(9):570-578 (August 30, 1990) and K. Culver et al., PNAS USA **88**:3155-3159 (April 1991)).

In another embodiment, minigenes encoding the mutant or wild-type p53 epitope are inserted into the eucaryotic cell in vitro using known techniques (see Hahn et al., Proc. Natl. Acad. Sci. USA **89**:2679-2683 (April 1992).

Suitable cloning/expression vectors for inserting DNA into eucaryotic cells include well-known derivatives of SV-40, adenovirus, cytomegalovirus (CMV), and retrovirus-derived DNA sequences. Any such vectors, when coupled with vectors derived from a combination of plasmids and phage DNA, i.e. shuttle vectors, allow for the cloning and/or expression of protein coding sequences in both procaryotic and eucaryotic cells.

Other eucaryotic expression vectors are known in the art, e.g., P.J. Southern and P. Berg, J. Mol. Appl. Genet. 1, 327-341 (1982); S. Subramani et al, Mol. Cell. Biol. 1, 854-864 (1981); R.J. Kaufmann and P.A. Sharp, "Amplification And Expression Of Sequences Cotransfected with A Modular Dihydrofolate Reductase Complementary DNA Gene," J. Mol. Biol. 159, 601-621 (1982); R.J. Kaufmann and P.A. Sharp, Mol. Cell. Biol. 159, 601-664 (1982); S.I. Scahill et al, "Expression and Characterization of the Product of a Human Immune Interferon DNA Gene in Chinese Hamster Ovary Cells," Proc. Natl. Acad. Sci. USA 80, 4654-4659 (1983); G. Urlaub and L.A. Chasin, Proc. Natl. Acad. Sci. USA 77, 4216-4220, (1980).

The wild-type p53 protein may also be presented to the immune system on the surface of recombinant bacterial cells. A suitable recombinant bacterial cell is an avirulent strain of Mycobacterium bovis, such as bacille Calmette-Guerin (BCG), or an avirulent strain of Salmonella, such as S. typhimurium. The recombinant bacterial cells may be prepared by cloning DNA comprising the active portion of the p53 protein in an avirulent strain, as is known in the art; see, for example, Curtiss et al., Vaccine 6, 155-160 (1988) and Galan et al., Gene 94, 29-35 (1990) for preparing recombinant Salmonella and Stover, K.S. et al., Vaccines 91, Cold Spring Harbor Laboratory Press, pp. 393-398 (1991) for preparing recombinant BCG.

Cloning vectors may comprise segments of chromosomal, non-chromosomal and synthetic DNA sequences. Some suitable prokaryotic cloning vectors include plasmids from E. coli, such as colE1, pCR1, pBR322, pMB9, pUC, pKSM, and RP4. Prokaryotic vectors also include derivatives of phage DNA such as M13, fd, and other filamentous single-stranded DNA phages.

Vectors for expressing proteins in bacteria, especially E.coli, are also known. Such vectors include the pK233 (or any of the tac family of plasmids), T7, and lambda P_{L}. Examples of vectors that express fusion proteins are PATH vectors described by Dieckmann and Tzagoloff in J. Biol. Chem. 260, 1513-1520 (1985). These vectors contain DNA sequences that encode anthranilate synthetase (TrpE) followed by a polylinker at the carboxy terminus. Other expression vector systems are based on beta-galactosidase (pEX); lambda P_{L}; maltose binding protein (pMAL); glutathione S-transferase (pGST) - see Gene 67, 31 (1988) and Peptide Research 3, 167 (1990).

The expression vectors useful in the present invention contain at least one expression control sequence that is operatively linked to the DNA sequence or fragment to be expressed. The control sequence is inserted in the vector in order to control and to regulate the expression of the cloned DNA sequence. Examples of useful expression control sequences are the lac system, the trp system, the tac system, the trc system, major operator and promoter regions of phage lambda, the control region of fd coat protein, and promoters derived from polyoma, adenovirus, retrovirus, and simian virus, e.g., the early and late promoters of SV40, and other sequences known to control the expression of genes in prokaryotic or eukaryotic cells and their viruses or combinations thereof.

The vaccine may further comprise pharmaceutically acceptable adjuvants, such as muramyl peptides, lymphokines, such as interferon, interleukin-1 and interleukin-6, or bacterial adjuvants. The adjuvant may comprise suitable particles onto which the wild-type p53 protein is adsorbed, such as aluminum oxide particles. These vaccine compositions containing adjuvants may be prepared as is known in the art.

An example of a bacterial adjuvant is BCG. When used as an antigen presenting cell as described above, recombinant BCG may additionally act as its own adjuvant. In this case, additional adjuvant may not be needed although one or more additional adjuvants may optionally be present. When used in its natural (non-recombinant) state, BCG acts solely as an adjuvant by being combined with wild-type p53, resulting in a form that induces an effective immune response.

The vaccine may also comprise a suitable medium. Suitable media include pharmaceutically acceptable carriers, such as phosphate buffered saline solution, liposomes and emulsions.

The invention further includes a method of inhibiting the growth of tumors in mammals comprising treating a mammal having a tumor or at imminent risk of obtaining a tumor with an immunologically effective amount of a vaccine comprising wild-type p53. A mammal is at imminent risk of obtaining a tumor if the mammal is diagnosed as having an abnormal, pre-cancerous condition.

The wild-type p53 is presented to the immune system of the mammal in a form that induces an effective immune response, i.e., either on the surface of an antigen presenting cell or combined with a pharmaceutically acceptable adjuvant. The wild-type p53 is preferably in a medium such as a pharmaceutically acceptable carrier.

The vaccine may be administered to a mammal by methods known in the art. Such methods include, for example, intravenous, intraperitoneal, subcutaneous, intramuscular, topical, or intradermal administration.

## Claims

1. A vaccine composition comprising a wild-type p53 protein, which is either the processed protein on the surface of an antigen presenting cell or the protein combined with a pharmaceutically acceptable adjuvant.

2. A vaccine composition according to claim 1 wherein the composition also comprises a pharmaceutically acceptable medium.

3. A vaccine composition according to claim 1, wherein the p53 protein is the processed protein on the surface of an antigen presenting cell.

4. A vaccine composition according to claim 1, wherein the p53 protein is the p53 protein combined with a pharmaceutically acceptable adjuvant.

5. A vaccine composition according to claim 3, wherein the antigen presenting cell is a eukaryotic cell.

6. A vaccine composition according to claim 5, wherein the eukaryotic cell is a dendritic cell, a major histocompatibility complex Class II positive macrophage or a monocyte.

7. A vaccine composition according to claim 6, wherein the antigen presenting cell is a dendritic cell.

8. A vaccine composition according to claim 7, wherein the dendritic cell is a recombinant dendritic cell that expresses exogenous DNA encoding wild-type p53 protein on its surface.

9. A vaccine composition according to claim 4, wherein the pharmaceutically acceptable adjuvant is a bacterial cell.

10. A vaccine composition according to claim 9, wherein the bacterial cell is bacille Calmette-Guerin.

11. A vaccine composition according to claim 10, wherein the bacille Calmette-Guerin is a recombinant bacille Calmette-Guerin that expresses exogenous DNA encoding wild-type p53 protein.

12. Use of a composition according to any of claims 1-11 for preparing a vaccine for inducing an effective immune response in a mammal.

13. A recombinant MHCII antigen presenting cell that expresses exogenous DNA encoding wild-type p53 protein.

## Patentansprüche

1. Impfstoffzusammensetzung umfassend ein Wildtyp p53-Protein, welches entweder als prozessiertes Protein auf der Oberfläche einer Antigen-präsentierenden Zelle vorliegt oder als Protein kombiniert mit einem pharmazeutisch annehmbaren Adjuvans.

2. Impfstoffzusammensetzung gemäß Anspruch 1, wobei die Zusammensetzung weiterhin ein pharmazeutisch annehmbares Medium umfasst.

3. Impfstoffzusammensetzung gemäß Anspruch 1, wobei das p53-Protein als prozessiertes Protein auf der Oberfläche einer Antigen-präsentierenden Zelle vorliegt.

4. Impfstoffzusammensetzung gemäß Anspruch 1, wobei das p53-Protein als p53-Protein kombiniert mit einem pharmazeutisch annehmbaren Adjuvans vorliegt.

5. Impfstoffzusammensetzung gemäß Anspruch 3, wobei die Antigen-präsentierende Zelle eine eukaryontische Zelle ist.

6. Impfstoffzusammensetzung gemäß Anspruch 5, wobei die eukaryontische Zelle eine dendritische Zelle, ein für den Major Histocompatibility Complex Klasse II positiver Makrophage oder ein Monozyt ist.

7. Impfstoffzusammensetzung gemäß Anspruch 6, wobei die Antigen-präsentierende Zelle eine dendritische Zelle ist.

8. Impfstoffzusammensetzung gemäß Anspruch 7, wobei die dendritische Zelle eine rekombinante dendritische Zelle ist, welche für Wildtyp p53-Protein kodierende exogene DNA auf ihrer Oberfläche exprimiert.

9. Impfstoffzusammensetzung gemäß Anspruch 4, wobei das pharmazeutisch annehmbare Adjuvans eine Bakterienzelle ist.

10. Impfstoffzusammensetzung gemäß Anspruch 9, wobei die Bakterienzelle eine Bacille Calmette-Guerin ist.

11. Impfstoffzusammensetzung gemäß Anspruch 10, wobei die Bacille Calmette-Guerin eine rekombinante Bacille Calmette-Guerin ist, welche für Wildtyp p53-Protein kodierende exogene DNA exprimiert.

12. Verwendung einer Zusammensetzung gemäß einem der Ansprüche 1-11 zur Herstellung eines Impfstoffes zur Induzierung einer effektiven Immunantwort in einem Säugetier.

13. Rekombinante MHCII-Antigen-präsentierende Zelle, welche für Wildtyp p53-Protein kodierende exogene DNA exprimiert.

## Revendications

1. Composition vaccinale comprenant une protéine p53 de type sauvage, qui est soit la protéine transformée sur la surface d'une cellule présentant un antigène, soit la protéine combinée à un adjuvant pharmaceutiquement acceptable.

2. Composition vaccinale selon la revendication 1, dans laquelle la composition comprend aussi un milieu pharmaceutiquement acceptable.

3. Composition vaccinale selon la revendication 1, dans laquelle la protéine p53 est la protéine transformée sur la surface d'une cellule présentant un antigène.

4. Composition vaccinale selon la revendication 1, dans laquelle la protéine p53 est la protéine p53 combinée à un adjuvant pharmaceutiquement acceptable.

5. Composition vaccinale selon la revendication 3, dans laquelle la cellule présentant un antigène est une cellule eucaryote.

6. Composition vaccinale selon la revendication 5, dans laquelle la cellule eucaryote est une cellule dendritique, un macrophage positif de classe. II du complexe majeur d'histocompatibilité ou un monocyte.

7. Composition vaccinale selon la revendication 6, dans laquelle la cellule présentant un antigène est une cellule dendritique.

8. Composition vaccinale selon la revendication 7, dans laquelle la cellule dendritique est une cellule dendritique recombinante qui exprime un ADN exogène codant une protéine p53 de type sauvage sur sa surface.

9. Composition vaccinale selon la revendication 4, dans laquelle l'adjuvant pharmaceutiquement acceptable est une cellule bactérienne.

10. Composition vaccinale selon la revendication 9, dans laquelle la cellule bactérienne est un bacille de Calmette-Guérin.

11. Composition vaccinale selon la revendication 10, dans laquelle le bacille de Calmette-Guérin est un bacille de Calmette-Guérin recombinant qui exprime un ADN exogène codant une protéine p53 de type sauvage.

12. Utilisation d'une composition selon l'une quelconque des revendications 1 à 11 pour préparer un vaccin destiné à induire une réponse immunitaire efficace chez un mammifère.

13. Cellule présentant un antigène MHCII recombinant qui exprime un ADN exogène codant une protéine p53 de type sauvage.
